# EUROPÄISCHE PATENTSCHRIFT

(11) **EP 3 730 158 B1**
(45) Veröffentlichungstag und Bekanntmachung des Hinweises auf die Patenterteilung: **12.01.2022**
(21) Anmeldenummer: 20000156.8
(22) Anmeldetag: 16.04.2020
(51) Int. Cl.: A61L 9/20, A61M 39/16, A61M 16/10, A61M 16/00, A61M 39/02, A61L 2/08

(54) **BEATMUNGSGERÄT**
BREATHING APPARATUS
APPAREIL RESPIRATOIRE

(30) Priorität: 25.04.2019 DE 102019110740
(43) Veröffentlichungstag der Anmeldung: 28.10.2020
(73) Patentinhaber: Löwenstein Medical Technology S.A., 2557 Luxembourg (LU)
(72) Erfinder: Schuster, Jan, 65824 Schwalbach am Taunus (DE)
(74) Vertreter: Marx, Thomas

(56) Entgegenhaltungen:
- WO-A1-2011/006206
- DE-A1- 2 347 791
- DE-A1- 10 107 443
- US-A- 3 126 003
- US-A1- 2006 177 356
- US-A1- 2007 163 588
- US-A1- 2008 032 119
- US-A1- 2018 104 374

## Beschreibung

Die Erfindung betrifft ein Beatmungsgerät. In der vorliegenden Erfindung wird unter einem Beatmungsgerät eine gesteuerte, beispielsweise elektronisch durch Mikroprozessoren, Vorrichtung verstanden, welche für eine Unterstützung oder Aufrechterhaltung oder zur therapeutischen Behandlung, beispielsweise CPAP-Therapie, der Atemfunktion eines Patienten ausgebildet ist. Hierbei sind auch solche Beatmungsgeräte eingeschlossen, welche als Narkosegeräte und/oder zur Beatmung von Patienten ohne Spontanatmung ausgebildet sind. Bei dem Patienten handelt es sich hierbei um einen Menschen oder um ein luftatmendes Tier, beispielsweise ein Säugetier.

Die Übertragung von Infektionskrankheiten durch Pathogene, das heißt, durch infektiöse Mikroorganismen wie beispielsweise Pilze, Pilzspören, Bakterien und Viren, erfolgt hauptsächlich über den Luftweg. In öffentlichen Gebäuden mit einem hohen Menschenaufkommen, insbesondere Krankenhäusern, ist in der Umgebungsluft die Konzentrationen an Pathogenen und damit die Infektionsgefahr besonders hoch. Menschen mit einem geschwächten Immunsystem sind hiervon besonders betroffen. Ein möglicher Infektionsweg führt hierbei über die durch Beatmungsgeräte applizierte oder beigemischte Luft aus Druckgasleitungen oder gefördert mittels eines Gebläses aus der Geräteumgebung.

Daher sind standardmäßig Beatmungsgeräte, auch solche, welche als Narkosegeräte ausgebildet sind, mit Partikelfiltern zur Abtrennung von Pathogenen versehen. Da diese Filter auf dem Prinzip des Größenausschlusses beruhen, sind hierbei nicht alle Pathogene abtrennbar. Bei solchen Beatmungsgeräten nach dem Stand der Technik ist also die Gefahr pneumonaler Infektionen nicht hinreichend gebannt.

Die desinfizierende Wirkung von ultravioletter Strahlung (UV) ist durch den österreichischen Arzt Gustav Kaiser seit 1902 bekannt, wobei ultraviolette Strahlung Wellenlängenbereiche von 100 nm bis 280 nm (UV-C), von 280 nm bis 315 nm (UV-B) und von 315 nm bis 380 nm (UV-A) umfasst. Beispielsweise ist in der US 2016 0271288 AI eine Vorrichtung beschrieben, welche für die Sterilisation von Luft mittels UV ausgebildet ist.
Eine weitere Vorrichtung zur Desinfektion von Mikroorganismen allgemein mittels polychromatischem Licht ist in der WO 2017 023783 A1 offenbart. Das polychromatische Licht weist hierbei einen signifikanten Wellenlängenanteil ultravioletter Strahlung auf, für dessen Erzeugung eine einzige Lichtquelle vorgesehen ist. Für die in WO 2017 023783 A1 beanspruchte Vorrichtung ist daher zur Ableitung thermischer Strahlung und zur Rückreflexion antimikrobiell wirksamer Strahlung ein dichroitischer Spiegel erforderlich.

Folglich hat es auch nicht an Vorschlägen gefehlt, die durch Beatmungsgeräte applizierte Atemluft oder Atemluftgemisch ebenfalls mittels UV von Pathogenen zu befreien, das heißt, zu desinfizieren. WO 2014 159874 A1 lehrt ein UV-Bestrahlungssystem, welches in medizinische Tubi, beispielsweise ein Trachealtubus, eingebrachte Lichtleiterfasern zur Abstrahlung von UV-Licht umfasst. Die WO 2012 052908 A1 offenbart ebenfalls einen Trachealtubus mit solchen Lichtleiterfasern, welche vorzugsweise elektromagnetische Strahlung im Wellenlängenbereich UV-C abgeben. Die WO 2015 092695 A1 umfasst ein Beatmungsgerät, in dessen pneumatischen Schaltungssystem eine Beleuchtungsvorrichtung zur Abgabe von allgemein desinfizierendem Licht integriert ist, wobei das desinfizierende Licht vorzugsweise durch UV und davon besonders bevorzugt durch UV-C gegeben und unter anderem durch Lichtquellen (LED) bereitgestellt ist. Die DE 101 07 443 A1 betrifft ein Verfahren zur Desinfektion von geförderter Atemluft bei Beatmungsgeräten mittels starker elektromagnetischer Strahlung innerhalb deren pneumatischen Schaltungssysteme, wobei die elektromagnetische Strahlung durch UV oder Mikrowellen gegeben und in Pulsform appliziert ist. Die CN 204233560 U beschreibt dagegen ein Narkosegerät mit desinfizierender Kondensatableitung, wobei komplementär zu den vorbenannten Beispielen nur der Ausatmungsschlauch sequentiell mit einem Ausatmungsrückschlagventil, einem Photokatalysator-Sterilisator und einer Kältefalle verbunden ist. Die CN 204233560 U offenbart zwar die Anwendung allgemein von UV zur Desinfektion, jedoch nicht die Beschaffenheit und Wirkungsweise des Photokatalysator-Sterilisators.

Weitere Geräte zur physikalischen Desinfektion der Einatemluft von Patienten bzw. allgemein der Luft durch Lichtstrahlung sind aus den Dokumenten US3126003A, DE2347791A1, US2007/163588A1, US2006/177356A1 und WO2011/006206A1 bekannt.

Beatmungsgeräte einschließlich Narkosegeräte nach dem Stand der Technik, welche zur Desinfektion geförderter Atemluft oder eines geförderten Atemluftgemisches mittels UV ausgebildet sind, weisen folgende wesentliche Nachteile auf:
- Die reine Bestrahlung der geförderten Atemluft oder eines geförderten Atemluftgemisches reicht für eine Abtötung von Pathogenen nicht aus, da hierfür die für eine Beatmung zu wählenden Flüsse viel zu hoch sind und somit die mit der geförderten Atemluft oder mit dem geförderten Atemluftgemisch transportierten Pathogene einer zu kurzen Bestrahlungsdauer ausgesetzt sind;
- Ultraviolette Strahlung (UV), insbesondere im Wellenlängenbereich UV-B und vor allem im Wellenlängenbereich UV-C schädigen bestrahlte Bauteile, vor allem solche aus Kunststoff.
- UV, insbesondere im Wellenlängenbereich UV-B und vor allem im Wellenlängenbereich UV-C verursacht in einem erheblichen Maße radikalische Dissoziationsreaktion von Inhalationsanästetika, insbesondere von halogenierten Derivaten wie Halothan, Isofluran, Sevofluran und Desfluran. Die gebildeten Radikale sind hierbei für den beatmeten Patienten hochtoxisch.
- Ultraviolette Strahlung (UV) im Wellenlängenbereich UV-B verursacht zusätzlich die Bildung von Singulett-Sauerstoff und Ozon aus dem Triplett-Sauerstoff der geförderten Atemluft oder des geförderten Atemluftgemisches. Singulett-Sauerstoff und Ozon sind ebenfalls für den beatmeten Patienten höchtoxisch.
- Aus dem Beatmungsgerät eventuell austretende ultraviolette Strahlung (UV) stellt sowohl für das Bedienungspersonal als auch für den Patienten eine erhebliche Gesundheitsgefahr dar.

Lichtquellen, als ein Beispiel ist eine Leuchtdiode genannt, sind kommerziell mit verschieden weiten Emissionsbereichen und Emissionsmaxima im Bereich UV bis Infrarot (IR) und in nahezu beliebigen Bauformen und Baugrößen erhältlich. Lichtquellen sind kostengünstig und weisen gegenüber anderen elektrischen Leuchtmitteln einen optimalen Wirkungsgrad auf. Jede Lichtquelle weist eine Emissionsfläche auf, welche zur Emission von Licht ausgebildet ist. Durch die Gestaltung der Form der Emissionsfläche ist somit eine Lichtemissionsrichtung als auch die geometrische Emissionsform des emittierten Lichtes einstellbar, beispielsweise als Lichtkegel oder als Lichtfächer. Lichtquellen emittieren jeweils polychromatisches Licht über ein Wellenlängenintervall und weisen innerhalb eines solchen Wellenlängenintervalls für eine bestimmte Wellenlänge mindestens ein Emissionsmaximum bezüglich der Intensität auf, wobei jeder Intensitätsverlauf bezüglich jeweils eines Emissionsmaximums jeweils einer Planck-Verteilung entspricht.

Die Aufgabe der Erfindung besteht darin, ein hinsichtlich der Hygiene und Betriebssicherheit gegenüber dem Stand der Technik verbessertes Beatmungsgerät und/oder einen entsprechenden Adapter zur Verfügung zu stellen.

Diese Aufgabe wird erfindungsgemäß durch das vorgeschlagene Beatmungsgerät mit den Merkmalen des Anspruches 1 bzw. dem vorgeschlagenen Adapter für ein Beatmingsgerät mit den Merkmalen des Anspruches 11 gelöst. Bevorzugte Ausführungsformen der Erfindung sind Gegenstand der abhängigen Ansprüche.

Das beanspruchte Beatmungsgerät weist ein pneumatisches Schaltungssystem zur Förderung von Beatmungsgas in einer Durchflussrichtung mittels einer Gasförderungsvorrichtung durch einen Geräteausgang über ein Interface zu einem Patienten auf. Die Gasförderungsvorrichtung ist durch ein Elektrogebläse zum Ansaugen von Außenluft oder durch einen Anschluss an eine Druckluftzuleitungsvorrichtung gegeben. In der vorliegenden Erfindung wird unter einem Interface eine Verbindungsvorrichtung verstanden, welche den Geräteausgang mit den Atmungsorganen des Patienten pneumatisch verbindet. Das Interface ist beispielsweise durch einen Beatmungsschlauch verbunden mit einem Trachealtubus gegeben, wobei der Trachealtubus direkt zu den Atmungsorganen des Patienten geführt ist. Beispielsweise ist der Beatmungsschlauch alternativ mit einer Beatmungsmaske verbunden, wobei die Beatmungsmaske umschließend auf Mund und/oder Nase des Patienten aufgesetzt und somit mit den Atmungsorganen des Patienten verbunden ist. Das pneumatische Schaltungssystem umfasst sämtliche Verrohrungen zum Durchfluss von Beatmungsgas, welche zwischen der Gasförderungsvorrichtung und dem Geräteausgang pneumatisch miteinander verbunden sind. Ist das beanspruchte Beatmungsgerät ausschließlich für die Beatmung eines Patienten ausgebildet, so besteht das Beatmungsgas aus natürlicher oder synthetischer Luft oder aus mit zusätzlichem Sauerstoff angereicherter natürlicher oder synthetischer Luft oder aus reinem Sauerstoff. Ist das beanspruchte Beatmungsgerät zusätzlich als Narkosegerät ausgebildet, so ist dem Beatmungsgas zumindest temporär mindestens ein Inhalationsanästhetikum beigemischt. In beiden Ausführungen des beanspruchten Beatmungsgerätes ist dem Beatmungsgas wahlweise zusätzlich gasförmiges und/oder aerosolisches Wasser und/oder ein oder mehrere Edelgase beigemischt. Hierbei ist die Durchflussrichtung des Beatmungsgases in Richtung von der Gasförderungsvorrichtung zu dem Geräteausgang festgelegt. Beim Betrieb des beanspruchten Beatmungsgerätes ist das Beatmungsgas typischerweise mit einem Fluss von bis zu 300 ml min⁻¹ mittels der Gasförderungsvorrichtung gefördert.

In dem beanspruchten Beatmungsgerät sind innerhalb des pneumatischen Schaltungssystems mehrere Partikelfilter mit in Durchflussrichtung jeweils abnehmender Porengröße angeordnet, auf welche mindestens eine Lichtquelle in einer Lichtemissionsrichtung ausgerichtet ist.

Ein Partikelfilter weist eine vordere Oberfläche und eine hintere Oberfläche auf, wobei der Partikelfilter in der Weise in dem pneumatischen Schaltungssystem pneumatisch geschaltet, dass beim Betrieb des beanspruchten Beatmungsgerätes der Fluss des Beatmungsgases in Durchflussrichtung zuerst die vordere Oberfläche und dann die hintere Oberfläche des Partikelfilters durchdringt. Somit entsprechen die vordere Oberfläche und die hintere Oberfläche im Wesentlichen oder ganz jeweils einem Strömungsquerschnitt. Eine solche Anordnung des Partikelfilters innerhalb des pneumatischen Schaltungssystems beruht auf einem **ersten erfinderischen Gedanken,** dass während des Flusses des Beatmungsgases durch den Partikelfilter Pathogene in ihrer Fließgeschwindigkeit abgebremst werden; hierbei ist es unerheblich, ob bei einem Fluss des Beatmungsgases durch den Partikelfilter die Pathogene vollständig oder teilweise oder gar nicht zurückgehalten werden. Allein durch die Abbremsung der Pathogene ist ein hinreichendes Zeitintervall gegeben, um durch Bestrahlung mit Licht die Pathogene teilweise oder vollständig abzutöten oder zumindest bezüglich ihrer Virulenz zu schwächen oder zu neutralisieren. Entscheidend ist also die Akkumulation von Pathogenen vor und auf der vorderen Oberfläche des Partikelfilters. Bei einem Durchdringen der hinteren Oberfläche durch Pathogene ist dagegen keine Akkumulation dieser gegeben, vielmehr resultiert ein Abtrag dieser durchgedrungenen Pathogene durch den Fluss des Beatmungsgases.

Ein **zweiter erfinderischer Gedanke** ist bei dem vorgeschlagenen Beatmungsgerät daher durch die Ausrichtung mindestens einer Lichtquelle in einer Lichtemissionsrichtung auf den Partikelfilter in der Weise realisiert, dass in Durchflussrichtung zuerst die Lichtquelle und dann nachfolgend der Partikelfilter fest angeordnet ist. Somit ist innerhalb des pneumatischen Schaltungssystems eine Abstrahlung von Licht auf die vordere Oberfläche des Partikelfilters mittels der Lichtquelle bewerkstelligt. Dies resultiert in einer Bestrahlung sowohl der vorderen Oberfläche akkumulierten als auch auf der vorderen Oberfläche abgeschiedenen Pathogene in Lichtemissionsrichtung der Lichtquelle. Beispielsweise ist bei entsprechender Fertigung des Partikelfilters aus einem zumindest teilweise transluzenten Material ein Eindringen des durch die Lichtquellen abgestrahlten Lichtes durch die vordere Oberfläche ermöglicht, so dass auch Pathogene innerhalb des Partikelfilters zumindest teilweise durch Bestrahlung erfasst sind.

Bei dem vorgeschlagenen Beatmungsgerät ist es keinesfalls erforderlich, dass die Lichtquelle innerhalb der Verrohrung des pneumatischen Schaltungssystems angebracht ist. Es ist lediglich erforderlich, dass die Lichtemissionsrichtung der Lichtquelle auf die vordere Oberfläche des Partikelfilters ausgerichtet und eine Bestrahlung der vorderen Oberfläche sichergestellt ist. Beispielsweise ist dies auch durch ein hinreichend transparentes Lichtfenster realisierbar, welches in die Verrohrung vor der vorderen Oberfläche des Partikelfilters eingelassen ist. Ein solches Lichtfenster ist beispielsweise aus Quarz oder Saphir gefertigt. Beispielsweise ist auf dem Lichtfenster die Lichtquelle in der Weise fixiert, dass in Lichtemissionsrichtung eine Bestrahlung der vorderen Oberfläche des Partikelfilters ermöglicht ist. Bei der Vermeidung der Anordnung der Lichtquelle innerhalb der Verrohrung ist somit zugleich die Ausbildung eines nachteiligen Strömungswiderstandes eliminiert, so dass besonders vorteilhaft innerhalb der Verrohrung eine Laminarströmung des Beatmungsgases realisierbar ist. Weiter ist eine solche Vorrichtung besonders vorteilhaft bei mehrstückiger Fertigung der Verrohrung und einer Halterung der Lichtquelle so gestaltbar, dass für eine einfache Wartung oder allfällige Reparatur des beanspruchten Beatmungsgerätes die Lichtquelle einfach abnehmbar ist, ohne dass ein Öffnen der Verrohrung erforderlich ist.

Das vorgeschlagene Beatmungsgerät beruht bezüglich des durch die Lichtquelle abgestrahlten Lichtes auf der Hierarchie gekoppelter Quantensysteme als **dritten erfinderischen Gedanken.** In Konsequenz daraus sind für jeden elektronischen Anregungszustand eines Moleküls oder eines Molekülaggregates jeweils ein Satz verschiedener Oszillatorzustände gegeben. Ein Pathogen, das heißt ein virulenter Mikroorganismus, entspricht prinzipiell einem Molekülaggregat. Wird durch eine elektronische Anregung durch Bestrahlung mit Licht, bei einem solchen Molekülaggregat ausgewählt aus einem ersten Wellenlängenbereich von 385 nm bis 780 nm, insbesondere aber aus einem ersten Wellenlängenbereich von 385 nm bis 425 nm, die Bindungsordnung einer chemischen Bindung herabgesetzt, so resultieren energetisch herabgesetzte Oszillatorzustände und damit energetisch herabgesetzte Dissoziationsenergien der betreffenden Bindung bei einem real gegeben Morse-Potential. Bei gleichzeitiger Einstrahlung von InfrarotStrahlung (IR) ausgewählt aus einem zweiten Wellenlängenbereich von mehr als 780 nm bis 2000 nm ist somit eine beschleunigte Dissoziation der betreffenden Bindung realisierbar. Es hat sich bei Pathogenen experimentell gezeigt, dass bei einer Bestrahlung mit Licht, welches Licht sowohl aus dem ersten Wellenlängenbereich als auch aus dem zweiten Wellenlängenbereich enthält, eine beschleunigte Abtötung oder zumindest eine beschleunigte Inaktivierung von Pathogenen bezüglich deren Virulenz erzielbar ist. Bei einer Bestrahlung mit Licht nur aus dem ersten Wellenlängenbereich *oder* nur aus dem zweiten Wellenlängenbereich jeweils *allein* resultiert dagegen in einer signifikant langsameren Abtötung oder signifikant langsameren Inaktivierung von Pathogenen.

Daher ist bei dem vorgeschlagenen Beatmungsgerät das auf den Partikelfilter abgestrahlte Licht aus einem *ersten Wellenlängenbereich* von 385 nm bis 780 nm (hinreichende Anregung von elektronischen Zuständen) *und* aus einem *zweiten Wellenlängenbereich* von mehr als 780 nm bis 1700 nm (entsprechend einer Anregung von Oszillatorzuständen) ausgewählt.

Nachfolgend wird auf vorteilhafte Weiterbildungen des vorgeschlagenen Beatmungsgerätes hinsichtlich einer Anordnung von Lichtquellen und deren Beschaffenheit näher eingegangen. Diese Weiterbildungen entsprechen schrittweisen Optimierungen des beanspruchten Beatmungsgerätes bezüglich Intensität und Effizienz der Beleuchtung des Partikelfilters.

In einer ersten Weiterbildung der Erfindung bezüglich der Lichtquellen ist jeweils beidseitig zu dem Partikelfilter mindestens eine Lichtquelle angeordnet. Somit ist auf den Partikelfilter auch auf dessen hintere Oberfläche mindestens eine Lichtquelle in deren Lichtemissionsrichtung ausgerichtet, wodurch besonders vorteilhaft eine Nachbehandlung von den Partikelfilter durchdringenden Pathogenen durch Bestrahlung mit Licht ermöglicht ist. Ist beispielsweise hierbei der Partikelfilter nicht nur aus einem transluzenten Material gefertigt, sondern weisen auch dessen vordere und dessen hintere Oberfläche eine hinreichend geringe Distanz zueinander auf, ist besonders vorteilhaft zusätzlich bei einer hinreichenden Bestrahlungsleistüng der Lichtquelle eine Bestrahlung über die gesamte Distanz zwischen hinterer und vorderer Oberfläche ermöglicht.

In einer zweiten Weiterbildung der Erfindung bezüglich der Lichtquellen ist der Partikelfilter auf Seite der Lichtquelle flächervollständig ausleuchtbar. Dies ist bereits jeweils mit einer Lichtquelle beispielsweise dadurch bewerkstelligbar, dass diese zur Emission von Licht in geometrischer Form eines Lichtkegels mit einem hinreichend großen Lichtkegelwinkel ausgebildet ist. Somit ist eine Bestrahlung aller Pathogene ermöglicht, welche beim Betreib des vorgeschlagenen Beatmungsgerätes auf die vordere Oberfläche des Partikelfilters und/oder durch dessen hintere Oberfläche mit dem Fluss des Beatmungsgases austreten.

In einer dritten Weiterbildung der Erfindung bezüglich der Lichtquellen ist der Partikelfilter umlaufend gleichmäßig mit mindestens einer Lichtquelle ausleuchtbar. Dies ist beispielsweise dadurch mit einer einzigen Lichtquelle bewerkstelligbar, indem deren Emissionsfläche selbst umlaufend, in sich geschlossen und nach innen abgewinkelt ausgebildet ist. Beispielsweise ist ein umlaufendes, in sich geschlossenes Lichtfenster in die Verrohrung eingelassen, über welches eine Lichtquelle mit einer umlaufenden, in sich geschlossenen und nach innen abgewinkelten Emissionsfläche geschoben und fixiert ist. Alternativ zu einer Lichtquelle sind beispielsweise mehrere Lichtquellen in jeweils gleichen Abständen zueinander um den Partikelfilter angeordnet. Durch eine gleichmäßige Ausleuchtung der vorderen und/oder hinteren Oberfläche des Partikelfilters ist gewährleistet, dass alle auf den Partikelfilter auftreffenden oder durch den Partikelfilter durchtretenden Pathogene einer gleichen oder zumindest annähernd gleichen Strahlungsdichte des durch die Lichtquelle oder Lichtquellen emittierten Lichtes ausgesetzt sind.

Mit einer Lichtquelle, welche beispielsweise zur Abstrahlung von Licht in einem Wellenlängenintervall von 385 nm bis 1700 nm, bevorzugt bis 2000 nm ausgebildet ist und nur ein einziges Emissionsmaximum aufweist, ist eine zumindest annähernd gleichmäßige Anregung sowohl von Elektronenzuständen als auch von Oszillatorzuständen nur dann erreichbar, wenn sich das Emissionsmaximum zwischen dem Wellenlängenbereich der Anregungen von Elektronenzuständen und dem Wellenlängenbereich der Anregungen von Oszillatorzuständen befindet. Für eine hinreichend hohe Anregung erfordert dies eine Lichtquelle mit einer sehr hohen Leistungsstärke.

Ist zur Bestrahlung des Partikelfilters nur eine Sorte von Lichtquellen zur Abstrahlung von Licht in einem Wellenlängenintervall von 385 nm bis 2000 nm vorgesehen und dementsprechend ausgebildet, so weisen Lichtquellen dieser Sorte daher vorzugsweise sowohl im ersten Wellenlängenbereich der Anregungen von Elektronenzuständen als auch im zweiten Wellenlängenbereich der Anregungen von Oszillatorzuständen jeweils mindestens ein Emissionsmaximum auf. Das heißt, dass eine Lichtquelle dieser Sorte über ein Wellenlängenintervall von 385 nm bis 2000 nm insgesamt mindestens zwei Emissionsmaxima aufweist. Mit Lichtquellen einer solchen Sorte ist schon mit einer vergleichsweisen geringen Leistungsstärke eine zumindest annähernd gleichmäßige Anregung sowohl von Elektronenzuständen als auch von Oszillatorzuständen und damit effiziente Abtötung und/oder Inaktivierung von Pathogenen erreichbar.

In einer vierten Weiterbildung der Erfindung bezüglich der Lichtquellen sind auf den Partikelfilter mehrere Sorten von Lichtquellen umlaufend in alternierender Reihenfolge bezüglich der Sorten von Lichtquellen ausgerichtet. Hierbei unterscheiden sich die Sorten von Lichtquellen durch das jeweils abgestrahlte Licht. Dies gestattet besonders vorteilhaft die Wahl und Kombination solcher Lichtquellen, deren jeweils abgestrahltes Licht bezüglich Wellenlängenintervall und/oder Emissionsmaximum auf die Anregung von Elektronenzuständen und/oder von Oszillatorzuständen abgestimmt ist. Somit ist es auch ermöglicht, die Beleuchtung des Partikelfilters bezüglich der Abtötung und/oder Inaktivierung spezifischer Pathogene, wie beispielsweise MRSA, gezielt anzupassen. Durch eine Anordnung verschiedener Sorten von Lichtquellen umlaufend um den Partikelfilter und in alternierender Reihenfolge ist flächenvollständige und gleichmäßige Ausleuchtung des Partikelfilters leicht realisierbar.,

In einer fünften Weiterbildung der Erfindung bezüglich der Lichtquellen ist eine Sorte solcher Lichtquellen gegeben, welche zur Abstrahlung mit einem Emissionsmaximum von 405 nm ausgebildet sind. Bezüglich der elektronischen Anregung einer großen Typenvielfalt von Pathogenen hat sich die Bestrahlung mit Licht einer Wellenlänge von 405 nm als Optimum erwiesen. Hierfür besonders leistungsfähige Lichtquellen sind zur Abstrahlung von Licht in einem auf 385 nm bis 425 nm begrenzten Wellenlängenintervall mit einem Emissionsmaximum von 405 nm ausgebildet.
In einer sechsten Weiterbildung der Erfindung bezüglich der Lichtquellen umfasst das abgestrahlte Licht aller Lichtquellen ein Wellenlängenkontinuum von 385 nm bis 2000 nm. Hierdurch ist die Anregung aller Elektronen und Oszillatorzustände sichergestellt. Dies ist beispielsweise durch drei Sorten von Lichtquellen realisiert, wobei jeweils
- die Lichtquellen der ersten Sorte zur Abstrahlung von Licht in einem auf 385 nm bis 425 nm begrenzten Wellenlängenintervall mit einem Emissionsmaximum von 405 nm,
- die Lichtquellen der zweiten Sorte zur Abstrahlung von Licht in einem auf 385 nm bis 1000 nm begrenzten Wellenlängenintervall mit einem Emissionsmaximum von 600 nm und
- die Lichtquellen der dritten Sorte zur Abstrahlung von Licht in einem auf 900 nm bis 2000 nm begrenzten Wellenlängenintervall mit einem ersten Emissionsmaximum von 1400 nm und einem zweiten Emissionsmaximum von 1600 nm ausgebildet sind.

Nachfolgend wird auf vorteilhafte Ausführungsformen des vorgeschlagenen Beatmungsgerätes hinsichtlich des Partikelfilters näher eingegangen. Das vorgeschlagene Beatmungsgerät ist keinesfalls auf einen in dem pneumatischen Schaltungssystem angeordneten Partikelfilter, insbesondere auf einen oder mehrere Partikelfilter von nur einer Sorte, beschränkt. Das vorgeschlagene Beatmungsgerät ist auch keinesfalls auf in dem pneumatischen Schaltungssystem angeordnete Partikelfilter beschränkt, auf welche jeweils mindestens eine Lichtquelle in einer Lichtemissionsrichtung ausgerichtet ist. Die Anzahl und Beschaffenheit der Partikelfilter ist allein durch eine zu wahrende Baugröße des beanspruchten Beatmungsgerätes und durch die Vorgabe eines maximalen gesamten Flusswiderstandes von beispielsweise bis zu 5 hPa min 120⁻¹ L⁻¹ festgelegt.

In einer ersten Ausführungsform der Erfindung bezüglich des Partikelfilters ist der Partikelfilter als Glasfritte oder als Pressling aus Glaswolle und/oder zum Rückhalt von Pathogenen ausgebildet. Eine Glasfritte oder ein Pressling aus Glaswolle ist besonders vorteilhaft durch Autoklavierung bei mehr als 130° C sterilisierbar und damit wiederverwendbar. Durch Wahl einer Porengröße des Partikelfilters ist eine Größenselektivität bezüglich des Rückhaltes von spezifischen Mikroorganismen und damit Pathogenen, beispielsweise Bakterien, Pilze und Viren, einstellbar. Durch Wahl eines Filtermaterials, beispielsweise Teflon oder Nylon oder eines hydrophoben oder hydrophilen Kunststoffes in Faserform, ist ebenfalls eine Typenselektivität des Partikelfilters bezüglich des Rückhaltes von spezifischen Mikroorganismen alternativ oder zusätzlich optimierbar. Partikelfilter verschiedenster Porengrößen und gefertigt aus verschiedensten Materialien zum Rückhalt von Partikeln und/oder Mikroorganismen sind im Handel kommerziell erhältlich. Durch einen dauerhaften Rückhalt von Partikeln und/oder Mikroorganismen in dem Partikelfilter ist die Desinfektion des Beatmungsgases zusätzlich verbessert.

In einer weiteren Variante der zweiten Ausführungsform der Erfindung bezüglich des Partikelfilters weist die Glasfritte eine Porenweite von 1 µm bis 200 µm, bevorzugt 2 bis 150 µm auf.

Eine solche Glasfritte weist keinen merklichen Flusswiderstand auf, ohne dass die Akkumulation von Mikroorganismen vor und auf der vorderen Oberfläche der Glasfritte beeinträchtigt ist.

Erfindungsgemäß sind in dem pneumatischen Schaltungssystem mehrere Partikelfilter mit in Durchflussrichtung jeweils abnehmender Porengröße angeordnet. Mit einer solchen kombinierten Anordnung ist ein Optimum an Verwendungsdauer der Partikelfilter gewährleistet, bis ein Auswechseln dieser aufgrund von Sättigung mit Partikeln und Mikroben erforderlich ist.

In manchen Ausführungsformen der Erfindung erfolgt die Energieversorgung der Lichtquellen über das Netzteil des Beatmungsgeräts. Dazu sind die Lichtquellen beispielsweise in das interne Stromnetz des Beatmungsgeräts integriert. Diese Integration ermöglicht beispielsweise auch den Betrieb der Lichtquellen in dem Fall, dass das Beatmungsgerät über einen Akku mit Energie versorgt wird.

In manchen Ausführungsformen erfolgt die Energieversorgung der Lichtquellen über eine - bezogen auf das Beatmungsgerät - externe Energiequelle. So können die Lichtquellen zum Beispiel mit einem eigenen Netzteil oder Akkus bzw. Batterien verbunden werden. Insbesondere für den erfindungsgemäßen Adapter ist die Verbindung mit einer externen Energieversorgung denkbar. Eine Verbindung der Lichtquellen mit einer externen Energiequelle ist aber auch bei Lichtquellen denkbar, welche in dem Beatmungsgerät verbaut sind.

In weiteren Ausführungsformen der Erfindung sind die Lichtquellen schaltbar, d.h. aktivierbar und deaktivierbar, eingerichtet. Beispielsweise sind die Lichtquellen mit einem Schalter verbunden, welcher die Energieversorgung zum Beispiel unterbrechen kann, die Lichtquellen also ausschalten bzw. deaktivieren kann. Ein solcher Schalter ist so eingerichtet, dass auch eine (erneute) Aktivierung bzw. ein Einschalten der Lichtquellen ermöglicht wird. Ein solcher Lichtschalter kann beispielsweise in das Gehäuse des Beatmungsgeräts integriert sein aber auch als extra Schalter ausgeführt sein. Der erfindungsgemäße Adapter verfügt beispielsweise über einen vom Beatmungsgerät unabhängigen Schalter.

In einer weiteren Ausführungsform sind die Lichtquellen so eingerichtet, dass sie durch eine nicht näher beschriebene Kontrolleinheit in ihrer Leuchtstärke variierbar (zum Beispiel durch Anpassen der Betriebsspannung der Lichtquellen) sind, also dimmbar sind. Darunter ist auch zu verstehen, dass die Leuchtstärke bzw. die Betriebsspannung der Lichtquellen soweit runtergeregelt werden kann, dass die Lichtquellen ausgeschaltet sind.

In einer Ausführungsform sind die Lichtquellen so eingerichtet und in das Beatmungsgerät integriert, dass diese durch ein Steuersystem des Beatmungsgeräts ein- und ausgeschalten werden können bzw. auch die Leuchtstärke variiert werden kann. Dadurch kann beispielsweise eine automatische Abschaltung der Lampen bei Umstellung des Beatmungsgeräts von Netz- auf Ackubetrieb eingerichtet werden.

Das beanspruchte Beatmungsgerät weist im Vergleich zu solchen nach dem Stand der Technik folgende Vorteile auf:
- Das durch die Lichtquellen abgestrahlte Licht ist bezüglich der Wellenlängen weder für das Fertigungsmaterial der Verrohrung noch für Menschen und Säugetiere schädlich.
- Bei dem vorgeschlagenen Beatmungsgerät entfallen damit technisch aufwändige Maßnahmen zum Schutz vor schädlicher elektromagnetischer Strahlung und zur Ableitung von überschüssiger Wärme.
- Bei einem abgestrahlten Licht in einem Wellenlängenbereich von 385 nm bis 2000 nm ist keine Bildung von toxischen Substanzen aus dem Beatmungsgas gegeben, selbst wenn dieses ein Inhalationsanästhetikum wie beispielsweise wie Halothan, Isofluran, Sevofluran und Desfluran enthält.
- Hinsichtlich einfacher Fertigung, wirtschaftlichem Betrieb, Hygiene und Betriebssicherheit ist das vorgeschlagene Beatmungsgerät gegenüber dem Stand der Technik wesentlich verbessert.

Die Bereitstellung eines Adapters für Beatmungsgeräte bietet darüber hinaus den Vorteil, Beatmungsgeräte ohne die beschriebenen Merkmale der Erfindung kostengünstig mit ebenjenen Merkmalen aufzurüsten. Der vorgeschlagene Adapter umfasst die gleichen Merkmale wie das vorgeschlagene Beatmungsgerät, kann dazu nachträglich oder zusätzlich, an ein Beatmungsgerät angebracht bzw. angeschlossen werden.

Die Begriffe Pathogene, Keime, Mikroorganismen werden in dieser Anmeldung als Synonyme verwendet, um insbesondere aber nicht ausschließlich beispielsweise Viren, Bakterien, Pilze zu bezeichnen.

Nachfolgend wird das vorgeschlagene Beatmungsgerät anhand einer Zeichnung näher erläutert. Hierin zeigt:
- Fig. 1: Das beanspruchte Beatmungsgerätes 1 in schematischer Teilansicht eines pneumatischen Schaltungssystems 2 im Längsschnitt;
- Fig. 2: Querschnitt durch das pneumatische Schaltungssystem 2.
- Fig. 3 - 5: Schematische Teilansicht des Beatmungsgerätes 1 mit Anordnung der Lichtquellen 41, 42, 43 im Bereich der Anzeigefläche 103.
- Fig. 6 - 8: Schematische Teilansicht der Atemgasabfuhr 102 mit Anordnung von Lichtquellen 41, 42, 43 in verschiedenen beispielhaften Ausführungsformen.
- Fig. 9: Querschnitt durch das pneumatische Schaltungssystem 2.
- Fig. 10: Schematische Teilansicht eines Patienteninterface 105 mit beispielhafter Anordnung der Lichtquellen 41, 42, 43

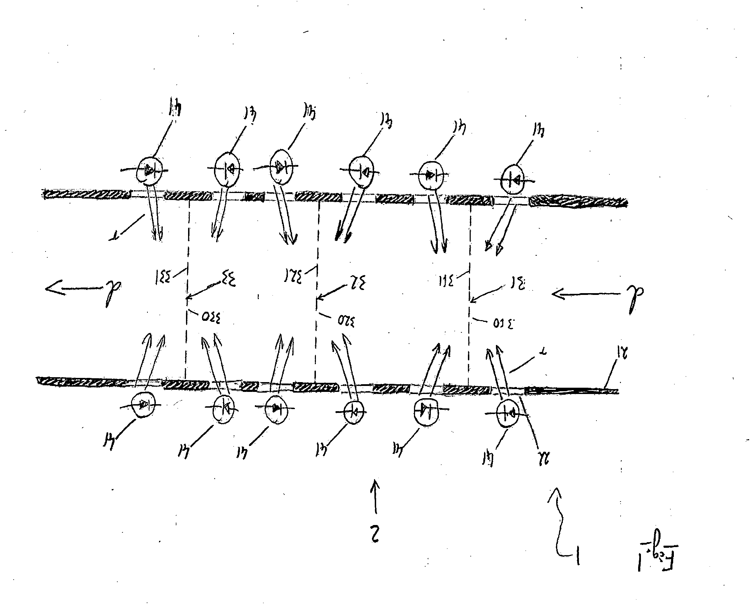

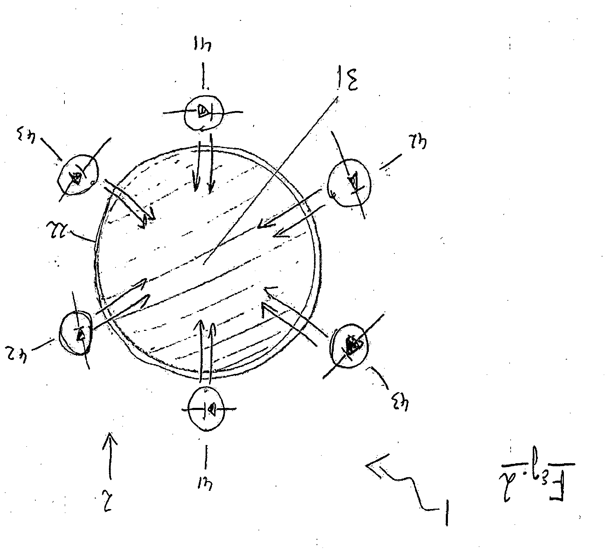

In dem nachfolgenden Ausführungsbeispiel ist das beanspruchte Beatmungsgerät 1 als Narkosegerät ausgebildet. Das Beatmungsgerät 1 weist ein pneumatisches Schaltungssystem 2 zur Förderung von Beatmungsgas in einer Durchflussrichtung d mittels eines Elektrogebläses durch einen Geräteausgang über ein Interface zu einem Patienten auf. Das Interface ist durch einen Beatmungsschlauch verbunden mit einem Trachealtubus gegeben, wobei der Trachealtubus direkt zu den Atmungsorganen des Patienten geführt ist. Die Fig. 1 und die Fig. 2 sind nicht maßstabsgetreu. In der Fig. 1 und in der Fig. 2 sind das Elektrogebläse, der Geräteausgang, das Interface, die Atmungsorgane des Patienten und der Patient nicht gezeigt. Zur Verdeutlichung des allgemeines Konstruktionsprinzips und Funktionsweise zeigen die Fig. 1 und die Fig. 2 jeweils schematische Teilansichten des beanspruchten Beatmungsgerätes 1.

Innerhalb des pneumatischen Schaltungssystems 2 sind drei Partikelfilter 31, 32 und 33 pneumatisch geschaltet. Beidseitig zu jedem Partikelfilter 31, 32 und 33 sind jeweils sechs Lichtquellen angeordnet, wobei die Lichtquellen solche einer ersten Sorte 41, einer zweiten Sorte 42 und einer dritten Sorte 43 umfassen. Auf die Partikelfilter 31, 32 und 33 sind die Lichtquellen 41, 42 und 43 in jeweils einer Lichtemissionsrichtung r ausgerichtet. In der Fig. 1 und in der Fig. 2 sind die Lichtemissionsrichtungen r jeweils in Form eines Doppelpfeils verdeutlicht.

Die Fig. 1 zeigt das beanspruchte Beatmungsgerät 1 in schematischer Teilansicht eines pneumatischen Schaltungssystems 2 im Längsschnitt. Innerhalb des pneumatischen Schaltungssystems 2 sind die ein bis drei Partikelfilter 31, 32 und 33 pneumatisch beispielsweise in einer zylindrischen Verrohrung 21 geschaltet. Die Partikelfilter 31, 32 und 33 weisen jeweils eine vordere Oberfläche 310, 320 und 330 und jeweils eine hintere Oberfläche 311, 321 und 331 auf, wobei die Partikelfilter 31, 32 und 33 in der Weise in dem pneumatischen Schaltungssystem 2 pneumatisch geschaltet sind, dass beim Betrieb des beanspruchten Beatmungsgerätes 1 der Fluss des Beatmungsgases in Durchflussrichtung d zuerst die jeweilige vordere Oberfläche 310, 320 und 330 und dann die jeweilige hintere Oberfläche 311, 321 und 331 des jeweiligen Partikelfilters 31, 32 und 33 durchdringt. Die Durchflussrichtung d ist in Fig. 1 durch einen Pfeil verdeutlicht. Somit entsprechen die vorderen Oberflächen 310, 320 und 330 und die hinteren Oberflächen 311, 321 und 331 im Wesentlichen jeweils einem Strömungsquerschnitt.

In die Verrohrung 21 sind insgesamt sechs Lichtfenster 22 über eine Nut-Feder-Verbindung fest eingelassen. Die Lichtfenster 22 sind ebenfalls jeweils als zylindrisches Rohrstück ausgebildet, welches neben zwei Verbindungsfedern auf jeder Anschlussseite denselben Radius und Manteldicke wie die Verrohrung 21 aufweist. Die somit umlaufenden, in sich geschlossenen Lichtfenster 22 sind in ihrer Bauform identisch und aus Quarz gefertigt. In Durchflüssrichtung d sind jeweils vor und jeweils nach jedem Partikelfilter 31, 32 und 33 ein Lichtfenster 22 angeordnet.

In der Fig. 1 sind nur die Lichtquellen der ersten Sorte 41 sichtbar. Auf jedem Lichtfenster 21 sind insgesamt sechs Lichtquellen 41, 42 und 43 in der Weise fixiert, dass jeweils in Lichtemissionsrichtung r eine Bestrahlung sowohl der jeweiligen vorderen Oberfläche 310, 320 und 330 als auch der hinteren Oberfläche 311, 321 und 331 des betreffenden Partikelfilters 31, 32 und 33 ermöglicht ist. In dem in Fig. 1 gezeigten Teilausschnitt der Verrohrung 21 ist somit eine Beeinflussung einer Laminarströmung des Beatmungsgases durch die Lichtquellen 41, 42 und 43 konstruktionsbedingt ausgeschlossen. In diesem Ausführungsbeispiel des beanspruchten Beatmungsgerätes 1 ist somit konstruktionsbedingt und besonders vorteilhaft eine Bestrahlung der Pathogene sowohl bei deren Akkumulation vor der vorderen Oberfläche 310, 320 und 330 als auch bei deren Durchtritt durch die hinteren Oberfläche 311, 321 und 331 der Partikelfilters 31, 32 und 33 ermöglicht.

Der Partikelfilter 31 ist beispielsweise als Glasfritte 31 ausgebildet. In die Glasfritte 31 ist beispielsweise zusätzlich Silber oder Titandioxid eingebracht, wobei das Titandioxid einen Massenanteil 1 % Platin aufweist. Somit ist die Glasfritte 31 als Katalysator ausgebildet, welcher die Oxidation von Mikroorganismen auf photo- als auch thermokatalytischem Wege beschleunigt; damit ist in diesem Ausführungsbeispiel eine Zersetzung, Abtötung und/oder Inaktivierung von Mikroorganismen auf drei voneinander unabhängige Arten realisierbar. Die Glasfritte 31 weist eine Porenweite von 1 µm bis 200 µm auf und stellt somit unter Betriebsbedingungen einen vernachlässigbaren Flusswiderstand dar. Der Partikelfilter 32 ist zum Rückhalt von Bakterien und Pilzen und der Partikelfilter 33 zum Rückhalt von Viren ausgebildet. Somit sind in dem pneumatischen Schaltungssystem 2 drei Partikelfilter 31, 32 und 33 mit in Durchflussrichtung d jeweils abnehmender Porengröße angeordnet.

Die Fig. 2 zeigt das beanspruchte Beatmungsgerät 1 in schematischer Teilansicht des pneumatischen Schaltungssystems 2 im Querschnitt. In Fig. 2 ist hierbei eine Aufsicht auf die vordere Oberfläche 310 des Partikelfilters 31gezeigt. Die Anordnungen der Lichtquellen 41, 42 und 43 um alle Partikelfilter 31, 32 und 33 sind jeweils identisch, so dass die Fig. 2 ist repräsentativ für die Anordnung aller Lichtquellen 41, 42 und 43 ist. Die in fig. 2 gezeigten Lichtquellen 41, 42 und 43 sind in jeweils gleichen Abständen zueinander und bezüglich einer Sorte gegenüberliegend um die vordere Oberfläche 310 des Partikelfilters 31 fest in einer gemeinsamen Halterung angeordnet. Dies entspricht einer Anordnung der Lichtquellen 41, 42 und 43 umlaufend in alternierender Reihenfolge bezüglich deren Sorten. Durch eine umlaufend gleichmäßige und flächenvollständige Ausleuchtung der vorderen Oberfläche 310 und hinteren Oberfläche 311 des Partikelfilters 31 ist gewährleistet, dass alle auf den Partikelfilter 31 auftreffenden oder durch den Partikelfilter 31 durchtretenden Pathogene einer gleichen oder zumindest annähernd gleichen Strahlungsdichte des durch die Lichtquellen 41, 42 und 43 emittierten Lichtes ausgesetzt sind. Die Halterung ist von der Verrohrung 21 zur einfacheren Wartung abnehmbar gestaltet. Somit ist jedem Lichtfenster 22 jeweils eine identische Halterung mit Lichtquellen 41, 42 und 43 zugeordnet. In diesem Ausführungsbeispiel sind:
- die Lichtquellen der ersten Sorte 41 zur Abstrahlung von Licht in einem auf 385 nm bis 425 nm begrenzten Wellenlängenintervall mit einem Emissionsmaximum von 405 nm,
- die Lichtquellen der zweiten Sorte 42 zur Abstrahlung von Licht in einem auf 385 nm bis 1000 nm begrenzten Wellenlängenintervall mit einem Emissionsmaximum von 600 oder 630 nm und
- die Lichtquellen der dritten Sorte 43 zur Abstrahlung von Licht in einem auf 900 nm bis 1700 oder 2000 nm begrenzten Wellenlängenintervall mit einem ersten Emissionsmaximum von 1300 oder 1400 nm und einem zweiten Emissionsmaximum von 1550 oder 1600 nm ausgebildet.

Hierdurch ist die Anregung aller Elektronen und Oszillatorzustände der Mikroorganismen sichergestellt.

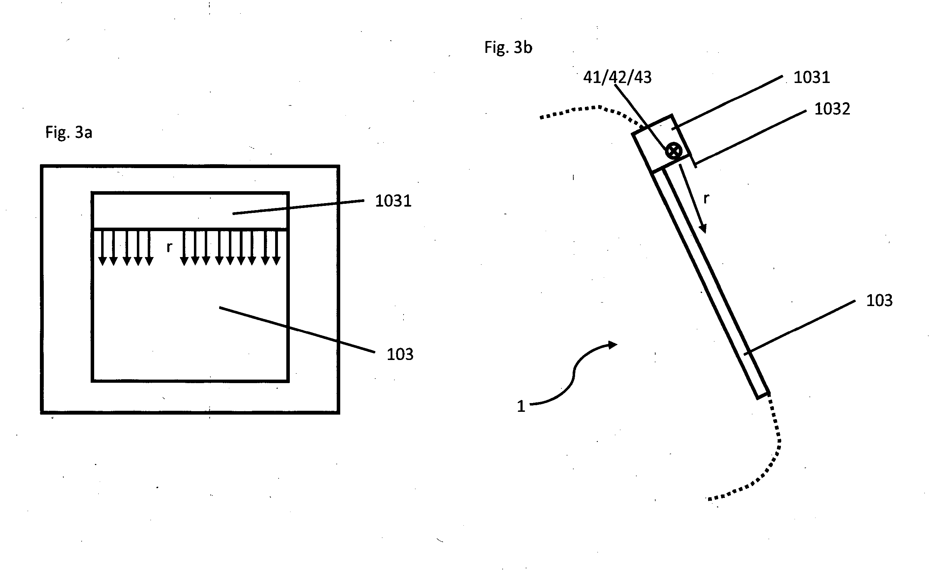

In Fig. 3a und 3b ist ausschnittsweise schematisch eine beispielhafte Ausführungsform des Beatmungsgeräts 1 gezeigt, bei dem die Lichtquellen 41, 42, 43 so angeordnet sind, dass die Anzeigefläche 103 bestrahlt wird. Die Lichtquellen 41, 42, 43 sind dazu beispielsweise in einem Lichtquellengehäuse 1031 angeordnet, sodass die Lichtquellen oberhalb der Anzeigefläche verortet sind. Somit ist es möglich, dass die Emissionsrichtung r der Lichtquellen 41, 42, 43 auf die Anzeigefläche 103 trifft. In einer beispielhaften Ausführung umfasst das Lichtquellengehäuse 1031 eine Art Schirm 1032, welche sich über das Ende der Lichtquellen hinaus erstreckt. Dieser Schirm 1032 bewirkt, dass zwar die Anzeigefläche 103 bestrahlt werden kann, die Lichtquellen 41, 42, 43 aber nicht in Richtung von beispielsweise auf die Anzeigefläche blickende Personen emittieren. In einer denkbaren weiteren beispielhaften Ausführung sind in dem Lichtquellengehäuse 1031 zusätzlich, nicht gezeigte, Reflektoren so angebracht, dass die Emissionsrichtung r der Lichtquellen 41, 42, 43 insbesondere auf die Anzeigefläche 103 gelenkt wird. Insbesondere wenn die Anzeigefläche 103 als Touchscreen ausgebildet ist, auf der die Eingaben, wie zum Beispiel Variation des Beatmungsdruckes, direkt auf der Anzeigefläche 103 eingestellt werden, ist es vorteilhaft, wenn diese Oberfläche durch die Lichtquellen sterilisiert werden kann.

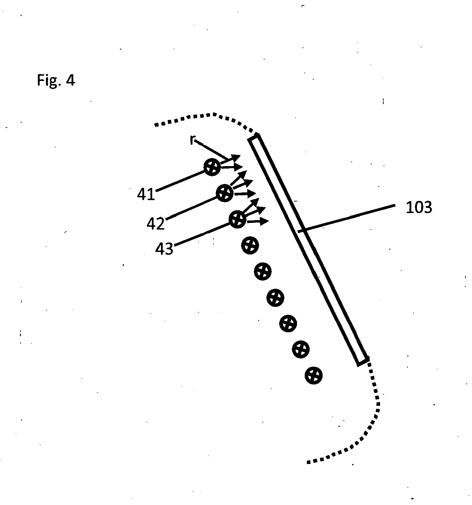

Die Fig. 4 zeigt schematisch einen Ausschnitt aus einer beispielhaften Ausführungsform des Beatmungsgeräts 1, bei dem die Lichtquellen 41, 42, 43 im Inneren des Beatmungsgeräts hinter der Anzeigefläche 103 angeordnet sind. Die Lichtquellen 41, 42, 43 sind so eingerichtet, dass die Emissionsrichtung r von hinten auf die Anzeigefläche strahlt. Vorteilhafter Weise ist die Anzeigefläche 103 beispielsweise so eingerichtet, dass die Strahlung durch die Anzeigefläche 103 durchscheint und zumindest die Oberfläche der Anzeigefläche erreicht. In einer beispielhaften Ausführung dienen die Lichtquellen 41, 42, 43 neben der Sterilisation der Oberfläche der Anzeigefläche auch gleichzeitig als Hintergrundbeleuchtung der Anzeigefläche 103. Dazu ist die Anzeigefläche 103 zum Beispiel als LCD ausgeführt.

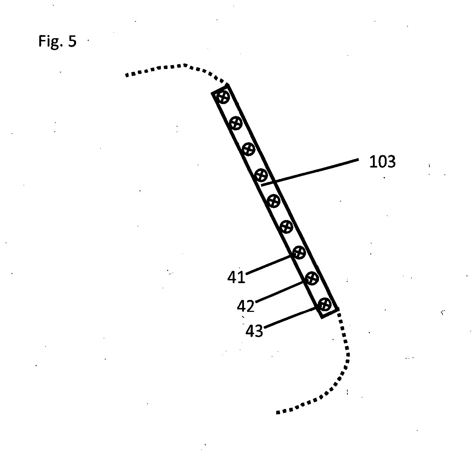

In Fig. 5 ist eine beispielhafte Ausführungsform des Beatmungsgeräts 1 ausschnittsweise gezeigt, bei dem die Lichtquellen 41, 42, 43 selbst Teil der Anzeigefläche 103 sind. Beispielsweise können die Lichtquellen als organische Leuchtdioden ausgeführt sein und zusammen ein OLED-Display ergeben. So können die allgemeinen Vorteile eines OLED-Displays - platzsparende Bauweise und geringer Strombedarf - mit der sterilisierenden Wirkung der gewählten Lichtquellen kombiniert werden.

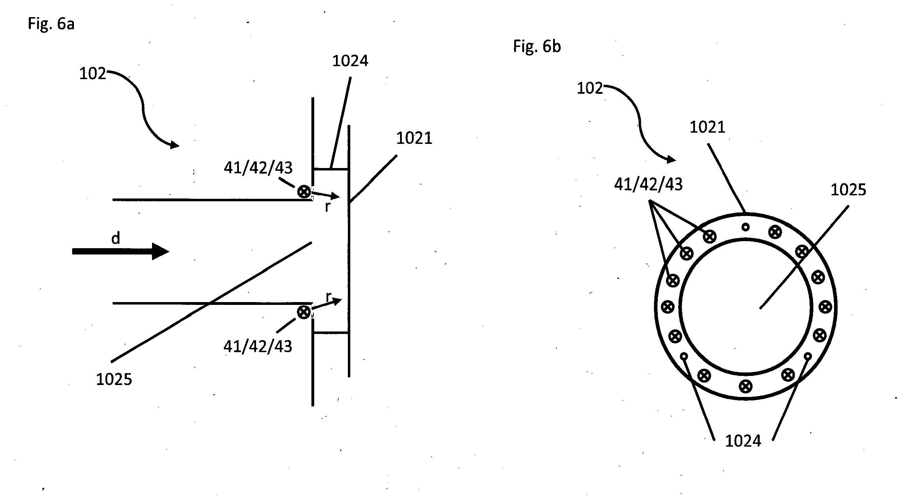

In den Figuren 6 bis 9 werden beispielhafte Ausführungsformen des Beatmungsgeräts 1 dargestellt, bei denen die Lichtquellen im Bereich der Atemgasabfuhr 102 angeordnet sind. Neben den gezeigten Beispielen sind weiterhin auch beispielsweise Kombinationen mit dem in Fig.1 und 2 beschriebenen Funktionsprinzip möglich. Die in Fig. 1 und 2 beschriebenen, beispielhaften Ausführungen der Teilabschnitte können zum Beispiel vor dem Ausgang 1025 innerhalb des Beatmungsgeräts 1 aber in Durchflussrichtung d nach dem Patienten angeordnet sein. Die in Figuren 6 bis 9 beschriebenen Ausführungsformen betreffen insbesondere Beatmungsgeräte, bei denen das Atemgas in beispielsweise einem zwei-Schlauchsystem vom Patienten zurück zum Beatmungsgerät geführt und dort durch zum Beispiel eine Atemgasabfuhr aus dem Beatmungssystem abgeführt werden.

Fig. 6a und 6b zeigen beispielhaft den Bereich der Atemgasabfuhr 102 des Beatmungsgeräts 1. Im Bereich der Atemgasabfuhr 102 ist beispielsweise eine Prallfläche 1021 angeordnet, welche den Ausgang 1025 der Atemgasabfuhr 102 abdeckt aber nicht verschließt. Dazu ist die Prallfläche 1021 durch Verbindungen 1024 in einem Abstand zu dem Ausgang 1025 der Atemgasabfuhr mit dem Bereich der Atemgasabfuhr 102 verbunden. Die Verbindungen 1024 sind beispielsweise beanstandet um den Ausgang 1025 angeordnet. Beispielhaft sind in Fig. 6b drei Verbindungen 1024 gezeigt, es könne aber auch mehr oder weniger Verbindungen zwischen der Prallfläche 1021 und dem Bereich der Atemgasabfuhr 102 angeordnet sein. Die Lichtquellen 41, 42, 43 sind beispielsweise im Bereich der Atemgasabfuhr 102 im Gehäuseinneren und um den Ausgang 1025 angeordnet. Die Lichtquellen 41, 42, 43 können dabei zum Beispiel so angeordnet sein, dass die einzelnen Typen der Lichtquellen alternierend angeordnet sind. Das heißt, dass neben einer Lichtquelle 41 eine Lichtquelle 42 und daneben eine Lichtquelle 43 angeordnet sein kann. Die Reihenfolge kann aber auch beliebig anders aufgebaut sein. Auch muss die Anzahl der einzelnen Lichtquellentypen nicht gleichmäßig verteilt sein - beispielsweise können doppelt so viele Lichtquellen 41 wie Lichtquellen 42 und 43 vorhanden sein. Auch können ausschließlich ein oder zwei Typen von Lichtquellen verwendet werden. Im Bereich der Atemgasabfuhr 102 weist das Gehäuse des Beatmungsgeräts 1 nicht näher beschriebene Öffnungen oder Lichtfenster auf, sodass die Lichtquellen 41, 42, 43 zum Beispiel in Richtung der Prallfläche . 1021 strahlen können. Die Fläche der Prallfläche 1021 ist beispielsweise größer als die Querschnittsfläche des Ausgangs 1025 und verdeckt den Ausgang vollständig, wie in Fig. 6b schematisch zu erkennen.

Die Prallfläche 1021 ist beispielweise so eingerichtet, dass das Beatmungsgas mit Durchflussrichtung d in Richtung der Prallfläche 1021 strömt und dabei auf die Prallfläche 1021 auftrifft. Dadurch wird zum einen die Strömungsgeschwindigkeit verlangsamt, zum anderen können sich so auch die Pathogene bzw. Keime aus dem Beatmungsgas auf der Fläche akkumulieren. Dadurch ist es möglich die Bestrahlungsdauer der Pathogene zu verlängern und führt zu einer effektiven Sterilisation. Beispielsweise kann die Prallfläche 1021 auch mit Silber oder platinhaltigem Titanoxid beschichtet sein um zusätzliche vorteilhafte Effekte, wie in Fig. 1 und 2 beschrieben, zu erreichen.

In Fig. 6a ist die Prallfläche 1021 beispielsweise senkrecht zur Durchflussrichtung d angeordnet. In anderen beispielhaften Ausführungen kann die Prallfläche 1021 auch geneigt zu der Durchflussrichtung d angeordnet sein. In weiteren Ausführungsformen ist die Prallfläche 1021 als strukturierte Oberfläche ausgebildet. In weiteren Ausführungsformen kann die Prallfläche 1021 auch andere geometrische Formen wie z.B. konisch, in Form einer Halbkugel oder eine beliebige Freiform annehmen. Es beispielsweise auch möglich, dass die Lichtquellen 41, 42, 43 nicht um den Ausgang 1025 verteilt sind, sondern nur stellenweise bzw. einseitig angeordnet sind.

In einer weiteren, nicht näher gezeigten Ausführungsform ist beispielsweise statt einer Prallfläche 1021 eine Anordnung von Partikelfiltern wie in Fig. 1 und Fig. 2 beschrieben im Bereich der Atemgasabfuhr 102 angeordnet sein.

Erfindungsgemäß können auch auf der Prallfläche Partikelfilter angeordnet sein. Die Partikelfilter sind in allen Ausführungsbeispielen eingerichtet und ausgebildet Keime (Viren, Bakterien, Pilze) zumindest teilweise zurück zu halten. Die sind in allen Ausführungsbeispielen beispielsweise zudem eingerichtet und ausgebildet das erfindungsgemäße Licht zumindest teilweise weiterzuleiten oder in tiefere Schichten des Filters eindringen zu lassen.

Es ist zu verstehen, dass die Anzahl und Anordnung der Lichtquellen sowie der Prallfläche hauptsächlich beispielhaft das Funktionsprinzip wiedergeben sollen und sowohl Lichtquellen als auch Prallfläche in verschiedener Anzahl, Anordnung und Form möglich ist.

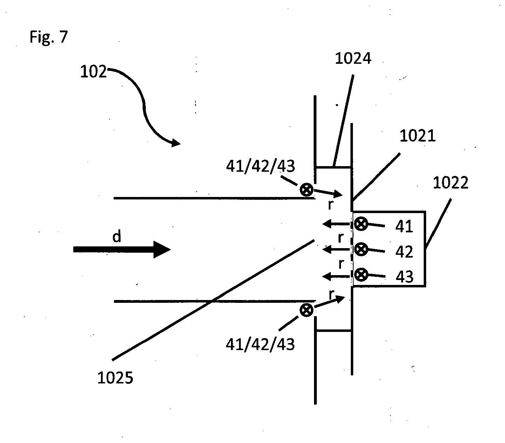

Fig. 7 zeigt einen Ausschnitt einer beispielhafte Ausführungsform des Beatmungsgeräts 1 im Bereich der Atemgasabfuhr 102. Die Grundelemente und Funktionsweise sind wie in Fig. 6 beschrieben. Zusätzlich sind beispielsweise noch auf der Prellfläche weitere Lichtquellen 41, 42, 43 in einem Lichtquellengehäuse 1022 angeordnet. Die Lichtquellen 41, 42, 43 sind so angeordnet und eingerichtet, dass die Emissionsrichtung r entgegen der Durchflussrichtung d ausgebildet ist. Dazu ist die Prallfläche 1021 so eingerichtet und ausgebildet, dass im Bereich der Lichtquellen Öffnungen oder vorzugsweise Lichtfenster angeordnet sind. Diese zusätzlichen Lichtquellen bieten den Vorteil, dass zum einen das Beatmungsgas (mit enthaltenen Pathogenen) bereits über eine Strecke vor dem Ausgang 1025 bestrahlt werden kann. Zum anderen verringert sich zusätzlich die Distanz zwischen den Lichtquellen und der Prallfläche 1021, sodass die Bestrahlungsintensität auf der Prallfläche 1021 zusätzlich erhöht werden kann oder alternativ auch eine schwächere Stärke der Lichtquellen 41,42, 43 genutzt werden kann.

In einer weiteren beispielhaften Ausführungsform ist es möglich auf die Lichtquellen auf Seite des Ausgangs 1025 zu verzichten und ausschließlich die Lichtquellen auf Seiten der Prallfläche 1021 anzuordnen.

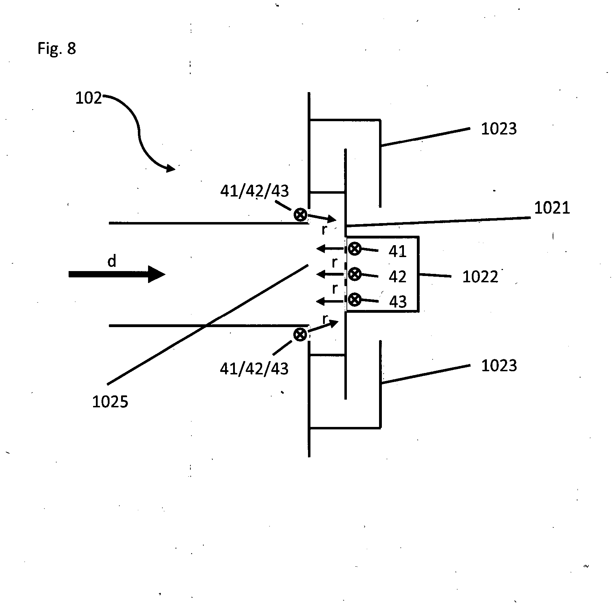

In Fig. 8 ist eine weitere beispielhafte Ausführungsform des Beatmungsgeräts 1 in einem Ausschnitt des Bereichs der Atemgasabfuhr 102 dargestellt. Zusätzlich zu den in Fig. 6a, b und Fig. 7 dargestellten beispielhaften Ausführungsformen, ist um die Prallfläche 1021 eine Art Mantel 1023 eingerichtet. Dieser Mantel 1023 ist beispielsweise mit dem Bereich der Atemgasabfuhr 102 verbunden und weist über der Prallfläche eine Öffnung auf. Der Mantel 1023 verhindert dabei, dass die Strahlung der Lichtquellen 41, 42, 43 auch außerhalb des Gerätes wahrgenommen werden können.

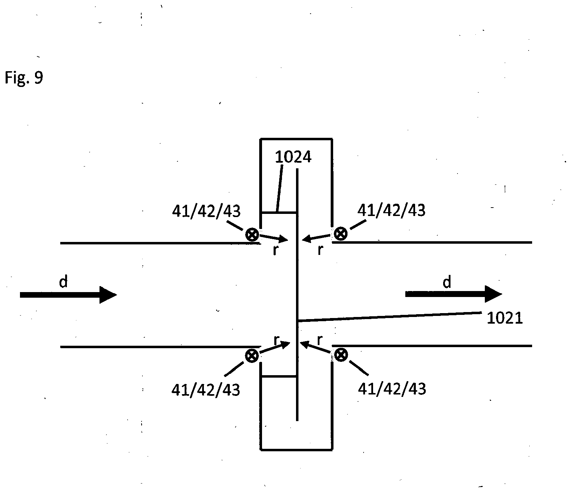

In Fig. 9 ist ein Ausschnitt aus einer weiteren beispielhaften Ausführungsform des Beatmungsgeräts 1 gezeigt. Die Lichtquellen 41, 42, 43 sind dabei an einer Stelle innerhalb des Gehäuses in Durchflussrichtung zwischen dem Patienten und dem Ausgang 1025 im Bereich der Atemgasabfuhr 102 angeordnet. Das generelle Funktionsprinzip der in Fig. 9 beispielhaft gezeigten Anordnung entspricht dem in Fig. 1 und 2 erläuterten Prinzip. Im Unterschied zu der in Fig.1 und 2 beschriebenen Ausführungsform wird hier lediglich eine Prallfläche 1021 in den Gasstrom eingeführt, welcher den Gasfluss verlangsamt und als Akkumulationsfläche für Pathogene dient.

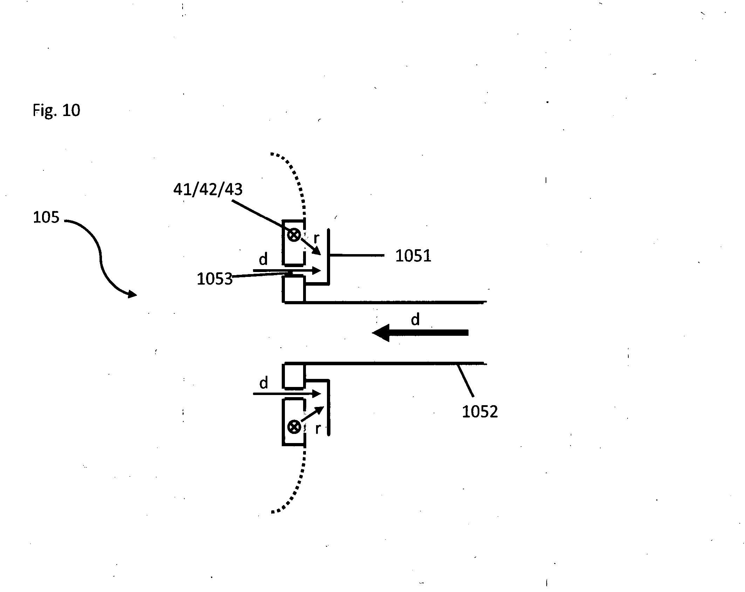

Fig. 10 zeigt einen beispielhaften Ausschnitt eines Patienteninterface 105 des Beatmungsgeräts 1. Die Lichtquellen 41, 42, 43 sind beispielhaft im Bereich der Ausströmöffnungen 1053 angeordnet. In Durchflussrichtung d nach den Ausströmöffnungen 1053 sind beispielsweise Abdeckungen 1051 angebracht, welche eine ähnliche bzw. die gleiche Funktion haben wie die in Fig. 6-9 beschriebenen Prallflächen 1021. Die Abdeckungen 1051 sind dabei zum Beispiel mit einem Teil des Patienteninterface 105 verbunden. Durch die Gaszuleitung 1052 wird dem Patienten das Beatmungsgas zugeführt. Das durch den Patienten ausgeatmete Beatmungsgas verlässt mit Durchflussrichtung d das Patienteninterface durch die Ausströmöffnungen 1053 und trifft dabei senkrecht auf die Abdeckung 1051. Die Abdeckung 1051 dient hier zum einen als Ort der Verlangsamung des Gasflusses aber auch zur mindestens temporären Akkumulation von Pathogenen bzw. Keimen, welche durch die Lichtquellen 41, 42, 43 bestrahlt und geschwächt bzw. inaktiviert werden. Die inaktivierten bzw. geschwächten Keime bzw. Pathogene werden regelmäßig durch den Gasstrom entfernt, sodass keine dauerhafte Akkumulierung eintritt.

### Bezugszeichenliste

- 1: Beatmungsgerät
- 101: Luftzufuhr
- 102: Atemgasabfuhr
- 103: Anzeigefläche
- 105: Patienteninterface
- 106: Gasflussleitung
- 107: Gasflussleitung
- 1021: Prallfläche
- 1022: Lichtquellengehäuse
- 1023: Mantel
- 1024: Verbindungen
- 1025: Ausgang
- 1031: Lichtquellengehäuse
- 1032: Schirm
- 1051: Abdeckung
- 1052: Gaszuleitung
- 1053: Ausströmöffnung
- 2: pneumatisches Schaltungssystem
- 21: Verrohrung
- 22: Lichtfenster
- 31: Partikelfilter
- 32: Partikelfilter
- 33: Partikelfilter
- 310: vordere Oberfläche
- 320: vordere Oberfläche
- 330: vordere Oberfläche
- 311: hintere Oberfläche
- 321: hintere Oberfläche
- 331: hintere Oberfläche
- 41: Lichtquelle einer ersten Sorte
- 42: Lichtquelle einer zweiten Sorte
- 43: Lichtquelle einer dritten Sorte
- 6: Adapter

- d: Durchflussrichtung
- r: Lichtemissionsrichtung

## Patentansprüche

1. Beatmungsgerät (1) mit einem pneumatischen System zur Förderung von Beatmungsgas in einer Durchflussrichtung (d) zu einem Patienten und optional von einem Patienten,
wobei das Beatmungsgerät (1) mindestens eine Lichtquelle (41, 42, 43) umfasst, wobei die mindestens eine Lichtquelle (41, 42, 43) in einer Emissionsrichtung (r) auf das Beatmungsgas und/oder auf das pneumatische System ausgerichtet ist und zumindest eine der Lichtquellen (41, 42, 43) dazu eingerichtet und ausgebildet ist, durch das abgestrahlte Licht Pathogene zumindest teilweise abzutöten und/oder zu inaktivieren, **dadurch gekennzeichnet, dass** das pneumatische System ein pneumatisches Schaltungssystem (2) zur Förderung von Beatmungsgas in einer Durchflussrichtung (d) zu dem Patienten aufweist und innerhalb des pneumatischen Schaltungssystems (2) mehrere Partikelfilter (31, 32, 33) mit in Durchflussrichtung (d) jeweils abnehmender Porengröße angeordnet sind und durch die Partikelfilter (31, 32, 33) Pathogene in ihrer Fließgeschwindigkeit zumindest abgebremst werden und wobei mindestens eine Lichtquelle (41, 42, 43) die dazu eingerichtet und ausgebildet ist, durch das abgestrahlte Licht Pathogene zumindest zu schwächen oder zu inaktivieren, in einer Lichtemissionsrichtung (r) auf zumindest einen Partikelfilter (31, 32, 33) ausgerichtet ist.

2. Beatmungsgerät (1) nach Anspruch 1, **wobei** abgestrahltes Licht aus einem ersten Wellenlängenbereich von 385 nm bis 780 nm und/oder aus einem zweiten Wellenlängenbereich von mehr als 780 nm bis 2000 nm ausgewählt ist um Pathogene zumindest zu schwächen oder zu inaktivieren.

3. Beatmungsgerät (1) nach zumindest einem der vorhergehenden Ansprüche, **wobei das** pneumatische System (2) zumindest eine Luftzufuhr (101) zum Beatmungsgerät (1) und/oder eine Atemgasabfuhr (102) des Patienten und/oder ein Patienteninterface (105) und/oder eine Gasflussleitung (106, 107) umfasst.

4. Beatmungsgerät (1) nach Anspruch 3, **wobei** mindestens eine Lichtquelle (41, 42, 43) im Bereich der Luftzufuhr (101) zum Beatmungsgerät (1) und/oder im Bereich der Atemgasabfuhr (102) des Patienten und/oder im Bereich des Patienteninterface (105) und/oder entlang der Gasflussleitungen (106, 107) angeordnet ist.

5. Beatmungsgerät (1) nach zumindest einem der vorhergehenden Ansprüche, **wobei** in Durchflussrichtung (d) zuerst die Lichtquelle (41, 42, 43) und nachfolgend der Partikelfilter (31, 32, 33) angeordnet sind und/oder jeweils beidseitig zu dem Partikelfilter (31, 32, 33) mindestens eine Lichtquelle (41, 42, 43) angeordnet ist.

6. Beatmungsgerät (1) nach zumindest einem der vorhergehenden Ansprüche, **wobei** der Partikelfilter (31, 32, 33) auf Seite der Lichtquelle (41, 42, 43) flächenvollständig ausleuchtbar ist.

7. Beatmungsgerät (1) nach zumindest einem der vorhergehenden Ansprüche, **wobei** der Partikelfilter (31, 32, 33) umlaufend gleichmäßig ausleuchtbar ist.

8. Beatmungsgerät (1) nach zumindest einem der vorhergehenden Ansprüche, **wobei** auf den Partikelfilter (31, 32, 33) mehrere Sorten von Lichtquellen (41, 42, 43) umlaufend in alternierender Reihenfolge bezüglich der Sorten von Lichtquellen (41, 42, 43) ausgerichtet sind, wobei sich die Sorten von Lichtquellen (41, 42, 43) durch das jeweils abgestrahlte Licht unterscheiden.

9. Beatmungsgerät (1) nach Anspruch 8, **wobei** das abgestrahlte Licht aller Lichtquellen (41, 42, 43) ein Wellenlängenkontinuum von 385 nm bis 2000 nm umfasst und/oder eine Sorte solcher Lichtquellen gegeben ist, welche zur Abstrahlung von Licht mit einem Emissionsmaximum von 405 nm ausgebildet ist.

10. Beatmungsgerät (1) nach zumindest einem der vorhergehenden Ansprüche, **wobei** der Partikelfilter (31) als Glasfritte (31) oder als Pressling aus Glaswolle und/oder zum Rückhalt von Pathogenen ausgebildet ist.

11. Adapter (6) für ein Beatmungsgerät (1) mit einem pneumatischen System (2) zur Förderung von Beatmungsgas, wobei das pneumatische System zumindest eine Luftzufuhr (101) zum Beatmungsgerät (1) und/oder eine Atemgasabfuhr (102) des Patienten und/oder ein Patienteninterface (105) und/oder eine Gasflussleitung (106, 107) und/oder Filter umfasst, wobei der Adapter (6) mit mindestens einer Lichtquelle (41, 42, 43) ausgestattet ist und mit der Luftzufuhr (101) und/oder der Atemgasabfuhr (102) und/oder dem Patienteninterface (105) und/oder einer Gasflussleitung (106, 107) verbunden werden kann, wobei die mindestens eine Lichtquelle (41, 42, 43) auf das Beatmungsgas und/oder das pneumatische System (2) ausgerichtet werden kann und die mindestens eine Lichtquelle (41, 42, 43) so eingerichtet und ausgebildet ist, durch das abgestrahlte Licht Pathogene zumindest zu schwächen oder zu inaktivieren, **dadurch gekennzeichnet, dass** der Adapter (6) mindestens eine Einrichtung (61) zur Verlangsamung der Strömung des Beatmungsgases und/oder zur zumindest temporären Akkumulierung und/oder Filterung von Pathogene aufweist, wobei die Einrichtung (61) in Form von mehreren mit in Durchflussrichtung (d) jeweils abnehmender Porengröße angeordneten Partikelfilter (31, 32, 33) ausgebildet ist und wobei mindestens eine Lichtquelle (41, 42, 43), die dazu eingerichtet und ausgebildet ist, durch das abgestrahlte Licht Pathogene zumindest zu schwächen oder zu inaktivieren, in einer Lichtemissionsrichtung (r) auf zumindest einen Partikelfilter (31,32,33) ausgerichtet ist.

12. Adapter (6) nach Anspruch 11, **wobei** die mindestens eine Einrichtung (61) in Form einer Prallfläche (1021) und/oder einer Abdeckung (1051) ausgebildet ist

## Claims

1. A breathing apparatus (1) comprising a pneumatic system for conveying breathing gas in a flow direction (d) to a patient and optionally from a patient, wherein the breathing apparatus (1) comprises at least one light source (41, 42, 43), wherein the at least one light source (41, 42, 43) is oriented in an emission direction (r) toward the breathing gas and/or toward the pneumatic system and at least one of the light sources (41, 42, 43) is designed and configured at least partially to extinguish and/or to inactivate pathogens by the radiated light, **characterized in that** the pneumatic system has a pneumatic circuit system for conveying breathing gas in a flow direction (d) to a patient, and a plurality of particle filters (31, 32, 33) with in each case a decreasing pore size in the flow direction (d) are arranged inside the pneumatic switching system (2), and pathogens are at least decelerated in their flow rate by the particle filters (31, 32, 33) and wherein at least one light source (41, 42, 43) which is designed and configured at least to weaken or to inactivate pathogens by the radiated light is oriented in a light emission direction (r) toward at least one particle filter (31, 32, 33).

2. The breathing apparatus (1) according to claim 1, wherein radiated light is selected from a first wavelength range of 385 nm to 780 nm and/or from a second wavelength range of more than 780 nm to 2000 nm at least in order to weaken or to inactivate pathogens.

3. The breathing apparatus (1) according to at least one of the preceding claims, wherein the pneumatic system (2) comprises at least one air supply (101) to the breathing apparatus (1) and/or a breathing gas outlet (102) of the patient and/or a patient interface (105) and/or a gas flow line (106, 107).

4. The breathing apparatus (1) according to claim 3, wherein at least one light source (41, 42, 43) is arranged in the region of the air supply (101) to the breathing apparatus (1) and/or in the region of the breathing gas outlet (102) of the patient and/or in the region of the patient interface (105) and/or along the gas flow lines (106, 107).

5. The breathing apparatus (1) according to at least one of the preceding claims, wherein firstly the light source (41, 42, 43) and subsequently the particle filter (31, 32, 33) are arranged in the flow direction (d) and/or in each case at least one light source (41, 42, 43) is arranged on either side of the particle filter (31, 32, 33).

6. The breathing apparatus (1) according to at least one of the preceding claims, wherein the particle filter (31, 32, 33) is able to be illuminated over the entire surface on the side of the light source (41, 42, 43).

7. The breathing apparatus (1) according to at least one of the preceding claims, wherein the particle filter (31, 32, 33) is able to be uniformly illuminated over the circumference.

8. The breathing apparatus (1) according to at least one of the preceding claims, wherein a plurality of types of light sources (41, 42, 43) are oriented toward the particle filter (31, 32, 33) in alternating sequence relative to the types of light sources (41, 42, 43) over the circumference, wherein the types of light sources (41, 42, 43) differ by the respectively radiated light.

9. The breathing apparatus (1) according to claim 8, wherein the radiated light of all of the light sources (41, 42, 43) comprises a wavelength continuum of 385 nm to 2000 nm and/or a type of such light sources which is configured for radiating light at a maximum emission of 405 nm is provided.

10. The breathing apparatus (1) according to at least one of the preceding claims, wherein the particle filter (31) is configured as a glass frit (31) or as a pressed part made of glass wool and/or for the retention of pathogens.

11. An adapter (6) for a breathing apparatus (1) comprising a pneumatic system (2) for conveying breathing gas, wherein the pneumatic system comprises at least one air supply (101) to the breathing apparatus (1) and/or a breathing gas outlet (102) of the patient and/or a patient interface (105) and/or a gas flow line (106, 107) and/or filter, wherein the adapter (6) is provided with at least one light source (41, 42, 43) and may be connected to the air supply (101) and/or the breathing gas outlet (102) and/or the patient interface (105) and/or a gas flow line (106, 107), wherein the at least one light source (41, 42, 43) may be oriented toward the breathing gas and/or the pneumatic system (2) and the at least one light source (41, 42, 43) is designed and configured so as at least to weaken or to inactivate pathogens by the radiated light, **characterized in that** the adapter (6) has at least one apparatus (61) for decelerating the flow of breathing gas and/or for at least temporarily accumulating and/or filtering pathogens, wherein the apparatus (61) is configured in the form of a plurality of particle filters (31, 32, 33) which are arranged in each case with a decreasing pore size in the flow direction (d) and wherein at least one light source (41, 42, 43) which is designed and configured at least to weaken or to inactivate pathogens by the radiated light is oriented in a light emission direction (r) toward at least one particle filter (31, 32, 33).

12. The adapter (6) according to claim 11, wherein the at least one apparatus (61) is configured in the form of a baffle (1021) and/or a cover (1051).

## Revendications

1. Appareil respiratoire (1) comprenant un système pneumatique destiné à transporter du gaz respiratoire dans une direction d'écoulement (d) vers un patient et optionnellement à partir d'un patient, dans lequel l'appareil respiratoire (1) comporte au moins une source de lumière (41, 42, 43), dans lequel l'au moins une source de lumière (41, 42, 43) est orientée vers le gaz respiratoire et/ou vers le système pneumatique dans une direction d'émission (r) et l'une au moins des sources de lumière (41, 42, 43) est agencée et conçue pour détruire et/ou inactiver au moins partiellement des pathogènes par le biais de la lumière rayonnée, **caractérisé en ce que** le système pneumatique présente un système de commutation pneumatique destiné à transporter du gaz respiratoire dans une direction d'écoulement (d) vers un patient et plusieurs filtres à particules (31, 32, 33) avec une taille de pores diminuant respectivement dans la direction d'écoulement (d) sont disposés à l'intérieur du système de commutation pneumatique (2) et des pathogènes sont au moins freinés dans leur vitesse d'écoulement par les filtres à particules (31, 32, 33) et dans lequel au moins une source de lumière (41, 42, 43) agencée et conçue pour au moins affaiblir ou pour inactiver des pathogènes par le biais de la lumière rayonnée est orientée vers au moins un filtre à particules (31, 32, 33) dans une direction d'émission de lumière (r).

2. Appareil respiratoire (1) selon la revendication 1, dans lequel de la lumière rayonnée est sélectionnée dans une plage première de longueur d'onde de 385 nm à 780 nm et/ou dans une deuxième plage de longueur d'onde de plus de 780 nm à 2000 nm pour au moins affaiblir ou pour inactiver des pathogènes.

3. Appareil respiratoire (1) selon l'une au moins des revendications précédentes, dans lequel le système pneumatique (2) comporte au moins une alimentation en air (101) vers l'appareil respiratoire (1) et/ou une évacuation de gaz de respiration (102) du patient et/ou une interface patient (105) et/ou un tube d'écoulement de gaz (106, 107).

4. Appareil respiratoire (1) selon la revendication 3, dans lequel au moins une source de lumière (41, 42, 43) est disposée dans la zone de l'alimentation en air (101) vers l'appareil respiratoire (1) et/ou dans la zone de l'évacuation de gaz de respiration (102) du patient et/ou dans la zone de l'interface patient (105) et/ou le long des tubes d'écoulement de gaz (106, 107).

5. Appareil respiratoire (1) selon l'une au moins des revendications précédentes, dans lequel la source de lumière (41, 42, 43) est disposée en amont et le filtre à particules (31, 32, 33) en aval dans la direction d'écoulement (d) et/ou au moins une source de lumière (41, 42, 43) est disposée respectivement de part et d'autre du filtre à particules (31, 32, 33).

6. Appareil respiratoire (1) selon l'une au moins des revendications précédentes, dans lequel le filtre à particules (31, 32, 33) peut être éclairé sur l'ensemble de sa surface du côté de la source de lumière (41, 42, 43).

7. Appareil respiratoire (1) selon l'une au moins des revendications précédentes, dans lequel le filtre à particules (31, 32, 33) peut être éclairé uniformément sur sa circonférence.

8. Appareil respiratoire (1) selon l'une au moins des revendications précédentes, dans lequel plusieurs types de sources de lumière (41, 42, 43) sont orientés circonférentiellement en alternance selon les types de sources de lumière (41, 42, 43) vers le filtre à particules (31, 32, 33), dans lequel les types de sources de lumière (41, 42, 43) se distinguent par la lumière respectivement rayonnée.

9. Appareil respiratoire (1) selon l'une au moins des revendications précédentes, dans lequel la lumière rayonnée de toutes les sources de lumière (41, 42, 43) comporte un continuum de longueur d'onde de 385 nm à 2000 nm et/ou il est fourni un type de ces sources de lumière conçu avec un pic d'émission de 405 nm pour le rayonnement de lumière.

10. Appareil respiratoire (1) selon l'une au moins des revendications précédentes, dans lequel le filtre à particules (31) est conçu comme une fritte de verre (31) ou comme un corps comprimé à base de laine de verre et/ou pour la retenue de pathogènes.

11. Adaptateur (6) pour un appareil respiratoire (1) comprenant un système pneumatique (2) pour le transport de gaz respiratoire, dans lequel le système pneumatique comporte au moins une alimentation en air (101) vers l'appareil respiratoire (1) et/ou une évacuation de gaz de respiration (102) du patient et/ou une interface patient (105) et/ou un tube d'écoulement de gaz (106, 107) et/ou un filtre, dans lequel l'adaptateur (6) est équipé d'au moins une source de lumière (41, 42, 43) et peut être relié à l'alimentation en air (101) et/ou à l'évacuation de gaz de respiration (102) et/ou à l'interface patient (105) et/ou au tube d'écoulement de gaz (106, 107), dans lequel l'au moins une source de lumière (41, 42, 43) peut être orientée vers le gaz respiratoire et/ou le système pneumatique (2) et l'au moins une source de lumière (41, 42, 43) est agencée et conçue pour au moins affaiblir ou pour inactiver des pathogènes par le biais de la lumière rayonnée, **caractérisé en ce que** l'adaptateur (6) présente au moins un dispositif (61) de ralentissement de l'écoulement du gaz respiratoire et/ou d'accumulation et/ou de filtration au moins temporaire de pathogènes, dans lequel le dispositif (61) est conçu sous la forme de plusieurs filtres à particules (31, 32, 33) disposés avec une taille de pores diminuant respectivement dans la direction d'écoulement (d) et dans lequel au moins une source de lumière (41, 42, 43) agencée et conçue pour au moins affaiblir ou pour inactiver des pathogènes par le biais de la lumière rayonnée est orientée vers au moins un filtre à particules (31, 32, 33) dans la direction d'émission de lumière (r).

12. Adaptateur (6) selon la revendication 11, dans lequel l'au moins un dispositif (61) est conçu sous la forme d'une surface d'impact (1021) et/ou d'un cache (1051).
